(19) Europäisches Patentamt
European Patent Office
Office européen des brevets

(11) Veröffentlichungsnummer: **0 025 961**
**B1**

(12) # EUROPÄISCHE PATENTSCHRIFT

(45) Veröffentlichungstag der Patentschrift: **25.05.83**

(51) Int. Cl.³: **C 07 C 29/10**, C 07 C 31/20, C 07 C 33/26, C 07 C 43/13, C 07 C 43/23

(21) Anmeldenummer: **80105510.4**

(22) Anmeldetag: **13.09.80**

(54) **Verfahren zur Herstellung von 1,2-Diolen höherer Kohlenstoffzahl.**

(30) Priorität: **21.09.79 DE 2938154**

(43) Veröffentlichungstag der Anmeldung: **01.04.81 Patentblatt 81/13**

(45) Bekanntmachung des Hinweises auf die Patenterteilung: **25.05.83 Patentblatt 83/21**

(84) Benannte Vertragsstaaten: **AT BE CH DE FR GB IT LI NL SE**

(56) Entgegenhaltungen:
**AT-B-299 142**
**DE-A-2 256 907**
**DE-B-2 109 453**

(73) Patentinhaber: **Henkel Kommanditgesellschaft auf Aktien, Postfach 1100 Henkelstrasse 67, D-4000 Düsseldorf 1 (DE)**
Patentinhaber: **Degussa Aktiengesellschaft, Weissfrauenstrasse 9, D-6000 Frankfurt am Main 1 (DE)**

(72) Erfinder: **Rutzen, Horst, Dr., Falkenweg 12, D-4018 Langenfeld (DE)**
Erfinder: **Rupilius, Wolfgang, Dr., Am Rittersberg 30, D-4000 Düsseldorf Urdenbach (DE)**

**EP 0 025 961 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

BUNDESDRUCKEREI BERLIN

# Verfahren zur Herstellung von 1,2-Diolen höherer Kohlenstoffzahl

Die Herstellung von 1,2-Diolen höherer Kohlenstoffzahl ist ein technisch noch nicht befriedigend gelöstes Problem. Ihre Gewinnung durch Hydrolyse der entsprechenden 1,2-Epoxidverbindung ist mehrfach vorgeschlagen worden, ohne daß eine in allen Punkten befriedigende Lösung gefunden worden wäre. Nachteilig bei den bisher bekannten Verfahren sind insbesondere der hohe Chemikalienverbrauch und/oder die hohen Temperaturen und die damit verbundenen hohen Drucke, die zur Erlangung befriedigender Ausbeuten notwendig sind.

Beispielsweise wird in Am. Soc. 68 (1946), 1504—1507, ein Verfahren vorgeschlagen, das von den entsprechenden Olefinen ausgeht und über die Bildung von Epoxiden verläuft. Hier wird ein großer Überschuß an Ameisensäure als Lösungsmittel und Sauerstoffüberträger (Peramiesensäure) benötigt. Der Chemikalienverbrauch wird zusätzlich dadurch erhöht, daß die zunächst entstehenden Diolester mit alkoholischer Kalilauge verseift werden.

Einen geringeren Chemikalienbedarf fordert das Verfahren der DE-OS 2 203 806, das zur Epoxidhydrolyse 2%ige Natronlauge verwendet. Nachteilig ist hier die hohe Reaktionstemperatur (250° C) und der hohe Druck (circa 40 atü), der aufwendige Druckapparaturen erforderlich macht. Der Austausch der Natronlauge gegen di- und monocarbonsaure Salze verbessert die Diolausbeuten, erfordert aber die gleiche hohe Reaktionstemperatur. In der DE-OS 2 256 907 wird vorgeschlagen, eine höhere Ausbeute durch Anwendung von Aceton als Lösungsvermittler und Dinatriumazelat als Spaltmittel zu erzielen. Auch in diesem Fall muß bei hohen Temperaturen (250° C) und entsprechenden Drucken gearbeitet werden. Die Säurehydrolyse mit verdünnter Schwefelsäure liefert neben circa 50% Diol erhebliche Mengen an Epoxidpolymeren.

Für die Hydrolyse kurzkettiger Epoxide (Ethylenoxid und Propylenoxid) wird in der DE-OS 2 615 595 vorgeschlagen, in Gegenwart von tert. Aminen (Triethylamin) und Kohlendioxid bei 110° C und 19 bar bei einer Hydrolysedauer von 2 Stunden zu arbeiten. Das kurzkettige Glykol entsteht mit hoher Selektivität. Die Übertragung dieser Reaktion auf Epoxidverbindungen höherer Kohlenstoffzahl ist bisher nicht gelungen.

Die Erfindung geht von der Aufgabe aus, die Nachteile und Einschränkungen der bisher bestehenden Verfahren zur Hydrolyse von 1,2-Epoxidverbindungen höherer Kohlenstoffzahl möglichst weitgehend zu vermeiden. Insbesondere soll die Herstellung der entsprechenden 1,2-Diole höherer Kohlenstoffzahl bei möglichst niedrigen Temperaturen und niedrigen Drucken innerhalb wirtschaftlich vertretbarer Reaktionszeiten zugänglich werden. Die Erfindung hat sich insbesondere die Aufgabe gesetzt, die an sich bekannte hydrolytische Spaltung von 1,2-Epoxidverbindungen durch Auswahl und Einsatz eines neuen Katalysatorsystems in Richtung auf einen optimalen Kompromiß von Reaktionsbedingungen und Ausbeute hin weiter zu entwickeln.

Gegenstand der Erfindung ist dementsprechend ein Verfahren zur Herstellung von 1,2-Diolen mit mindestens 4 Kohlenstoffatomen durch Spaltung der entsprechenden 1,2-Epoxidverbindungen mit Wasser in Gegenwart von Katalysatoren bei erhöhten Temperaturen. Das erfindungsgemäße Verfahren ist dadurch gekennzeichnet, daß man als Katalysatoren Salze von primären, sekundären und/oder tertiären Aminen mit organischen und/oder anorganischen Säuren und/oder quartäre Ammoniumsalze organischer und/oder anorganischer Säuren einsetzt.

Bevorzugt wird erfindungsgemäß mit Katalysatorsystemen auf Basis von Salzen der primären, sekundären und/oder tertiären Amine mit ein- oder mehrbasischen Carbonsäuren gearbeitet.

Als Epoxidverbindungen höherer Kohlenstoffzahl sind insbesondere entsprechende Ausgangsmaterialien geeignet, die 4 bis 30 Kohlenstoffatome enthalten, wobei Verbindungen mit etwa 6 bis 20 Kohlenstoffatomen besondere technische Bedeutung zukommen kann. Das erfindungsgemäße Verfahren ist zur Umwandlung entsprechender aliphatischer Epoxide, ebenso aber auch zur Umwandlung aromatischer Epoxide geeignet, vorausgesetzt, daß die eingesetzten Epoxidverbindungen wenigstens eine endständige Epoxidgruppe — die 1,2-Epoxidgruppierung — aufweisen. Das erfindungsgemäße Verfahren ist insbesondere auch anwendbar auf solche Epoxidverbindungen, die mehr als nur eine endständige Epoxygruppierung besitzen.

Epoxidverbindungen der genannten Art leiten sich beispielsweise von entsprechenden $\alpha$-Olefinen oder endständig ungesättigten araliphatischen Verbindungen ab. Für die Zwecke der Erfindung eignen sich aber als Ausgangsmaterialien insbesondere auch entsprechende Oxaverbindungen, d. h. strukturanaloge Komponenten, in denen ein die 1,2-Epoxygruppe enthaltender Molekülbestandteil über ein Sauerstoffatom an den höheren Kohlenwasserstoffrest gebunden ist. Besonders leicht zugänglich sind in diesem Fall die entsprechenden Glycidether, wobei auch hier die Glycidgruppierung ein- oder mehrfach im Ausgangsmaterial gebunden vorliegen kann.

Die für das erfindungsgemäße Verfahren besonders geeigneten Ausgangsmaterialien lassen sich unter der allgemeinen Formel

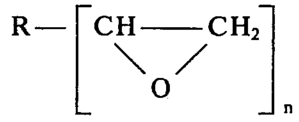

zusammenfassen, worin der Rest R ein offenkettiger, cyclischer, gesättigter oder ungesättigter, gewünschtenfalls auch aromatischer Kohlenwasserstoffrest mit 2 bis 28 Kohlenstoffatomen, vorzugsweise 4 bis 18 Kohlenstoffatomen und insbesondere 8 bis 18 Kohlenstoffatomen ist. An Stelle der reinen Kohlenwasserstoffreste können auch vergleichbare Reste mit Heteroatomen, beispielsweise entsprechende heterocyclische Reste vorliegen. In der angegebenen allgemeinen Formel bedeutet n eine vorzugsweise ganze Zahl, die gleich oder größer 1 ist und beispielsweise bis zu 5, vorzugsweise 1 bis 3, betragen kann. Besonders wichtig können Verbindungen sein, in denen n den Wert von 1 besitzt, und davon wiederum solche, in denen R ein offenkettiger, gesättigter Kohlenwasserstoffrest mit 8 bis 18 Kohlenstoffatomen ist.

Lassen sich die Epoxidverbindungen von endständigen Olefinen ableiten, dann ist die 1,2-Epoxygruppe in der Regel ausschließlich mittels Kohlenstoffbindungen mit dem Rest R verbunden. In dem zuvor geschilderten Fall der entsprechenden Oxaverbindungen ist als Bindeglied zwischen dem den Epoxidring tragenden Molekülbestandteil und dem Rest des Moleküls eine —O-Brücke vorgesehen. Im erfindungsgemäß besonders bevorzugten Fall der Glycidverbindungen hat der Rest R aus der zuvor angegebenen allgemeinen Formel dann die Bedeutung

$$R'-\!\!\left[O-CH_2\right]_{n'}$$

worin R′ die Bedeutung von R hat und n′ gleich n ist. Das erfindungsgemäße Verfahren eignet sich insbesondere zur hydrolytischen Spaltung von 1,2-Epoxidverbindungen, die auf Grund ihrer höheren Kohlenstoffzahl beziehungsweise ihrer Molekülstruktur mit Wasser nur beschränkt mischbar sind. Die Verwendung der im folgenden geschilderten neuen Katalysatorsysteme ermöglicht eine wirkungsvolle hydrolytische Spaltung unter vergleichsweise mäßigen Verfahrensbedingungen gerade für diese Stoffklasse und macht eine Optimierung der Produktausbeute unter diesen Bedingungen möglich.

Die erfindungsgemäß bevorzugten Katalysatoren beziehungsweise Katalysatorsysteme sind aus primären, sekundären und/oder tertiären Aminen als eine Komponente und salzbildenden anorganischen oder organischen Säuren als andere Komponente aufgebaut. Die bevorzugten Säuren sind ein- oder mehrbasische Carbonsäuren.

Die basische Komponente und die saure Komponente werden zweckmäßigerweise wenigstens in stöchiometrisch etwa gleichen Mengenverhältnissen eingesetzt. In einer bevorzugten Ausführungsform wird allerdings mit einem Überschuß der sauren Komponente über die zur Salzbildung hinaus benötigte Menge gearbeitet. Schon ein leichter Überschuß kann sich in einer beachtlichen Reaktionsbeschleunigung und Ausbeuteverbesserung unter Standardbedingungen bemerkbar machen. Die saure Komponente kann in einem mehrfachen — beispielsweise dem zwei- bis dreifachen — derjenigen Menge eingesetzt werden, die zur Salzbildung mit der basischen Komponente stöchiometrisch benötigt wird. Im allgemeinen wird allerdings bereits die gewünschte Reaktionsbeschleunigung mit Katalysatorsystemen erzielt, die die saure Komponente bis zum Doppelten der zur Salzbildung stöchiometrisch benötigten Menge enthalten. Besonders bevorzugt kann es sein, mit stöchiometrischen Verhältnissen von Base zu Säure im Bereich von 1 : 1,1 bis 1,8, insbesondere 1 : 1,1 bis 1,5, zu arbeiten.

Dieses Katalysatorsystem wird in begrenzten Mengen dem Reaktionsgemisch zugesetzt. Geeignet sind beispielsweise Mengen von 0,5 bis 10 Molprozent, insbesondere 1 bis 6 Molprozent, des Aminsalzes, bezogen auf eingesetztes Epoxid.

Eine Reaktionsbeschleunigung kann grundsätzlich mit beliebigen primären, sekundären und/oder tertiären Aminen als basische Komponente erzielt werden. Zur Optimierung des Verfahrensergebnisses ist allerdings zweckmäßig, eine Auswahl der einzusetzenden basischen Aminkomponenten zu treffen. Diese Auswahl bestimmt sich in einer bevorzugten Ausführungsform nach der Kohlenstoffzahl der Amine und ihrer Anpassung an die Kohlenstoffzahl der umzusetzenden Epoxidverbindungen. Für die drei Klassen der primären, der sekundären und der tertiären Amine gelten dabei die folgenden Auswahlkriterien:

Primäre Amine:

Zweckmäßigerweise beträgt die Summe der Kohlenstoffatome des primären Amins + dem 3fachen der Summe der Kohlenstoffatome der umzusetzenden Epoxidverbindung 8—40. Besonders bevorzugt liegt diese Summe von Kohlenstoffatomen im Zahlenbereich von 12—30.

Sekundäre Amine:

Hier beträgt die Summe der Kohlenstoffatome des sekundären Amins + dem Doppelten der Kohlenstoffatome des umzusetzenden Epoxids ebenfalls 8—40. Auch hier liegt in der besonders bevorzugten Ausführungsform der Zahlenwert dieser Summe von Kohlenstoffatomen im Bereich von 12—30.

Tertiäre Amine:

Entsprechend gilt für diese Stoffklasse, daß die Summe der Kohlenstoffatome des tertiären Amins + der Summe der Kohlenstoffatome des Epoxids wieder im Bereich von 8—40 liegt, wobei

auch hier der bevorzugte Zahlenwert 12 – 30 beträgt.

Diese Auswahl der Amine scheint eine Verteilung des Katalysatorsystems im heterogenen Reaktionsgemisch unter Reaktionsbedingungen besonders zu begünstigen, womit ein Zugang zur Optimierung des Verfahrensergebnisses eröffnet wird. Dabei scheinen die Amine unter dem Einfluß der sauren Katalysatorkomponente einer Alkylierung und Quaternierung durch Umsetzung mit den Epoxiden zu unterliegen.

Bevorzugte Amine der genannten Art sind aliphatische, cycloaliphatische und araliphatische Amine. Es können aber auch aromatische Amine zum Einsatz kommen. Die Verwendung von kurzkettigen Aminen kann zu besonders günstigen Ergebnissen führen. Kurzkettige aliphatische Amine und hier insbesondere sekundäre Amine dieser Art können besonders vorteilhafte Ergebnisse liefern. Dialkylamine mit bis zu 8 Kohlenstoffatomen, vorzugsweise mit bis zu 4 Kohlenstoffatomen sind eine bevorzugte basische Komponente für die erfindungsgemäß eingesetzten Katalysatorsysteme. Ein typisches Beispiel ist hier das Dimethylamin. Weitere wichtige Amine sind in den nachfolgenden Beispielen geschildert. Die Amine können unsubstituiert oder substituiert sein, unsubstituierte Amine sind im allgemeinen bevorzugt. Liegen Substituenten vor, so sollte durch sie die Basizität der Amine nicht zu stark beeinträchtigt werden. Geeignete Substituenten sind beispielsweise Hydroxylgruppen, sofern sie in nicht zu großer Häufung am Amin vorliegen. Allgemein bevorzugt können sekundäre Amine sein.

Die erfindungsgemäß bevorzugt zur Salzbildung eingesetzten Mono- und/oder Polycarbonsäuren besitzen 1 bis 26 Kohlenstoffatome. In Betracht kommen grundsätzlich organische Säuren aus der aliphatischen, der aromatischen und der heterocyclischen Reihe, wobei diese Säuren substituiert oder unsubstituiert und gesättigt oder ein- oder mehrfach ungesättigt sein können.

Typische Beispiele für Carbonsäuren der genannten Art sind aliphatische Monocarbonsäuren des genannten C-Zahlbereichs, insbesondere solche mit 2 bis 18, vorzugsweise 2 bis 14 Kohlenstoffatomen, die gegebenenfalls auch olefinisch ungesättigt sind. Aliphatische Di- und höhere Polycarbonsäuren — die ebenfalls gewünschtenfalls olefinisch ungesättigt sein können — liegen im C-Zahlbereich von 2 bis 26. Beispiele für substituierte Carbonsäuren sind Hydroxycarbonsäuren, Chlorcarbonsäuren, Dichlorcarbonsäuren, Trichlorcarbonsäuren. Geeignete aromatische Carbonsäuren sind beispielsweise Benzoesäure oder Zimtsäure sowie die aromatischen Dicarbonsäuren von der Art der Phthalsäure, Isophthalsäure oder Terephthalsäure. In Betracht kommen weiterhin heterocyclische Carbonsäuren wie Brenzschleimsäure, Tetrahydrofurancarbonsäuren und ähnliche Verbindungen.

Durch die Auswahl der Säure im Katalysatorsystem ist eine weitere Steuerungsmöglichkeit zur Optimierung des Verfahrensergebnisses eröffnet. Kurzkettige Säuren, beispielsweise Essigsäure, fördern die Wasserlöslichkeit des Katalysatorsystems, längerkettige Säuren erhöhen dessen Oleophilie.

Geeignet als saure Katalysatorkomponente sind aber auch anorganische Säuren. Hier sind insbesondere die Phosphorsäure und deren Partialsalze neben Amidosulfonsäure, Salpetersäure, Salzsäure und Schwefelsäure zu nennen.

Für ein technisches Verfahren geeignete Optimierungen konnten beispielsweise mit Katalysatorsystemen auf Basis Dimethylamin/Essigsäure oder Dimethylamin/Laurinsäure erzielt werden. Auch hier zeigen die Ausführungsbeispiele weitere besonders geeignete Kombinationen.

Werden dem Reaktionsgemisch von vornherein quartäre Ammoniumsalze als Katalysatoren zugesetzt, so besitzen auch diese eine deutliche katalytische Wirkung. Als Beispiele sind hier Tetraalkylammoniumsalze oder Aralkyltrialkylammoniumsalze zu nennen. Der Säurerest in diesen quartären Salzen kann anorganischer oder organischer Natur sein. Geeignet sind beispielsweise die quartären Ammoniumhalogenidsalze, in gleicher Weise können aber auch organische Säuren der zuvor genannten Art den Säurerest des Quartärsalzes darstellen. Da — wie zuvor geschildert — sich unter den Reaktionsbedingungen auch die Aminsalze vermutlich durch Reaktion mit den Epoxiden zu quartären Salzen umsetzen, leuchtet die Brauchbarkeit quartärer Ammoniumsalze als Katalysatoren für das erfindungsgemäße Verfahren ein. Geeignete Katalysatoren dieser Art sind beispielsweise Methyltrioctylammoniumchlorid, Quartärsalze, die am Ammoniumstickstoff zwei langkettige und zwei kurzkettige aliphatische Alkylreste oder einen langkettigen und zwei kurzkettige aliphatische Reste sowie einen araliphatischen Rest enthalten. Geeignet sind aber auch von Hydroxyaminen abgeleitete Quartärprodukte.

Werden solche Quartärsalze als Katalysatoren eingesetzt, so kann es erfindungsgemäß bevorzugt sein, zusätzlich zum getrennt vorgebildeten Quartärsalz beschränkte Mengen an saurer Komponente mit in das Verfahren einzusetzen. Hier gelten die zuvor gemachten Angaben zum Ausmaß des Säureüberschusses über die zur Salzbildung mit der basischen Aminkomponente benötigte Säuremenge. Als saure Komponenten kommen hier alle zuvor genannten anorganischen und insbesondere organischen Säuren in Betracht.

Bezüglich der zusammen mit dem Epoxid einzusetzenden Wassermenge besteht nach oben keine prinzipielle Einschränkung. Es ist jedoch ein wichtiger Vorteil des erfindungsgemäßen Verfahrens, daß mit einem nur beschränkten Wasserüberschuß gearbeitet werden kann und gleichwohl hohe

Ausbeuten an gewünschtem Verfahrensprodukt entstehen. In der Regel wird die Wassermenge 10 Mol Wasser pro Mol Epoxid nicht überschreiten, wobei schon mit Mengen bis zu 5 Mol die gewünschten Umsetzungen erhalten werden können. Der Mindestbetrag an Wasser entspricht dem theoretischen Bedarf von 1 Mol pro Mol Epoxid, üblicherweise wird mit Mengen von 1 bis 3 Mol Wasser pro Mol Epoxid gearbeitet. Durch diese Möglichkeit der Einschränkung der Wassermenge wird die Wirtschaftlichkeit des Verfahrens begünstigt.

Die Reaktionstemperatur liegt üblicherweise bei wenigstens 100°C. Geeignet ist vor allem der Bereich von 100—180°C. Es kann wünschenswert sein, als obere Reaktionstemperatur 160°C nicht zu überschreiten. Durch Abstimmung der Verfahrensvariablen und insbesondere durch Auswahl des geeigneten Katalysatorsystems können selbst bei so niedrigen Temperaturen wie 120°C oder gar bei 100°C befriedigende Ausbeuten an 1,2-Diolen in annehmbaren Zeiträumen erhalten werden. Als besonders wichtiger Temperaturbereich kann das Arbeiten im Bereich von 100—150°C gelten.

Die Verfahrensdrucke richten sich nach der gewählten Verfahrenstemperatur. Druckloses Arbeiten beim Siedepunkt des Wassers ist bei geeigneter Auswahl hochaktiver Katalysatorsysteme möglich. Andere Katalysatoren fordern höhere Temperaturen und damit erhöhte Drucke. Der geeignete Druckbereich liegt üblicherweise zwischen 1 und 10 bar, vorzugsweise im Bereich von 1 bis 6 bar. Dabei kann das Arbeiten im geschlossenen System bei autogenem Druck besonders bevorzugt sein.

Die Dauer der hydrolytischen Spaltung liegt üblicherweise im Bereich einiger Stunden, bevorzugt ist eine Behandlung von 2 bis 10 Stunden. Das Reaktionsgemisch sollte dabei bewegt werden. Wird in geschlossenen Reaktoren gearbeitet, so können hier Rühr- beziehungsweise Schüttelautoklaven eingesetzt werden.

Das Verfahrensprodukt fällt häufig als Emulsion an, aus der kaum eine Abtrennung des überschüssigen Wassers möglich ist. Eine Reinigung des Reaktionsproduktes vom überschüssigen Wasser kann durch einfaches Abdestillieren des Wassers im Vakuum erfolgen. Auf diese Weise bleibt der Katalysator im Reaktionsprodukt. Bei vielen Anwendungszwecken stören diese geringen Mengen des Katalysators nicht. Gewünschtenfalls kann der Katalysator in konventioneller Weise vom Reaktionsprodukt abgetrennt werden.

1,2-Diole besitzen ein ausgedehntes Anwendungsgebiet. Sie sind in Cremes und Hautbehandlungsmitteln anwendbar und gelten als besonders hautfreundlich, da sie im Wollfett vorkommen. Sie werden ebenso wie ihre Borsäurederivate als Textilweichmacher und in Form ihrer Carbonsäurederivate als Schaumregulatoren in Wasch- und Reinigungsmitteln vorgeschlagen.

Die Oxethylate der 1,2-Diole sind ausgezeichnete nichtionische Tenside mit sehr guten Wascheigenschaften. Sie sind ähnlich vielseitig einsetzbar wie die Fettalkoholoxethylate.

Für den technischen Einsatz können Diole eines Kettenlängenbereiches von 10 bis 20 Kohlenstoffatomen und hier insbesondere des Bereiches von 12 bis 16 Kohlenstoffatomen besonders wichtig sein. Erfindungsgemäß wird es möglich, Diole dieser Art — beispielsweise ein technisches 12/14-Diol-Gemisch bei Temperaturen bis zu etwa 135°C und Drucken nicht über 3 bar mit hohen Ausbeuten herzustellen.

## Beispiele

### Beispiele 1—30

In den folgenden Beispielen 1 bis 30 sind — nach Gruppen geordnet — Verfahrensergebnisse nach der Lehre der Erfindung zusammengefaßt. Bewußt wird dabei darauf verzichtet, in jedem Einzelfall eine Optimierung der Verfahrensbedingungen in Richtung auf eine hohe Ausbeute vorzunehmen.

Die erste Gruppe der Beispiele 1—8 bezieht sich auf die Verwendung verschiedener Amine. In dieser Versuchsreihe wurde technisches destilliertes Dodecenoxid und als zweite Katalysatorkomponente Essigsäure verwendet. Die Reaktionsbedingungen dieser Versuchsgruppe liegen bei 160°C, 7 bar und vierstündiger Behandlungsdauer.

Die zweite Versuchsgruppe (Beispiele 9 bis 12) beschreibt die Reaktion von Epoxiden unterschiedlicher Kettenlänge. Als Katalysator wurde N,N-Dimethyl-2-hydroxydodecylamin-Essigsäure eingesetzt. Die Reaktionsbedingungen waren 160°C, 7 bar bei 6-stündiger Behandlungsdauer.

Die dritte Versuchsgruppe (Beispiele 13—18) beschäftigt sich mit der Verwendung verschiedener Säuren als zweite Katalysatorkomponente. Als Einsatzmaterial dient technisches, destilliertes Dodecenoxid, als Aminkomponente wird Trimethylamin eingesetzt. Die Reaktionsbedingungen liegen hier bei 120°C, 3 bar und 4 Stunden Reaktionsdauer.

In der vierten Gruppe (Beispiele 19—27) werden Kombinationen von längerkettigen Aminen mit längerkettigen Carbonsäuren eingesetzt. Das Epoxidausgangsmaterial ist Dodecenoxid. Die Reaktionsbedingungen sind 100°C und Normaldruck bei vierstündiger Reaktionsdauer. Selbst bei diesen milden Reaktionsbedingungen werden hohe Produktausbeuten erhalten, die praktisch durchweg über 40% liegen und bis zu 60% erreichen. Durch leichte Verschärfung der Reaktionsbedingungen (längere Behandlungszeit und/oder Steigerung von Temperatur und Druck) können die in der Praxis benötigten hohen Ausbeuten an gewünschtem Endprodukt erhalten werden.

Die letzte Gruppe (Beispiele 28—30) befaßt sich mit der Ausprüfung von Quartärsalzen bei 160°C, 7 bar und vierstündiger Behandlungsdauer an Dodecenoxid als Ausgangsmaterial.

Die Versuche der Beispiele 1—30 wurden dabei wie folgt durchgeführt:

Das Olefinoxid wird zusammen mit Wasser und dem Katalysator in einen Rührautoklaven gefüllt und für die Dauer der angegebenen Reaktionszeit auf die angegebene Temperatur erhitzt. Das Reaktionsgemisch wird aus dem Autoklaven in eine normale Vakuumdestillationsapparatur überführt und zunächst im Wasserstrahlvakuum (circa 12 Torr) bis zu einer Sumpftemperatur von 110°C getrocknet. Anschließend erfolgt die Destillation im Ölpumpenvakuum bei circa 2 mbar unter Auftrennung im Vorlauf, Hauptlauf, Nachlauf und Rückstand. Vom Vorlauf wird der EpO-Wert und vom Hauptlauf der EpO und die OHZ bestimmt. Die geringen Katalysatormengen werden nicht abgetrennt, sondern in der Substanz belassen. Die Ergebnisse sind in der Tabelle 1 zusammengestellt.

Tabelle 1

| Beispiel | Epoxid Art | Reinh. | %EpO | Katalysator Aminkomponente Art | Menge g | Säurekomponente Art | Menge g | Endprodukte Vorlauf % | %EpO | Hauptlauf % | %EpO | OHZ | Nachl. % | Rückst. % |
|---|---|---|---|---|---|---|---|---|---|---|---|---|---|---|
| 1 | 1 Mol α-C12 | techn. dest. | 8,23 | 4-(Dimethylamino)-pyridin | 5 | Essigsäure | 3,1 | 3,2 | 0,40 | 90,2 | 0,02 | 537 | 3,9 | 8,1 |
| 2 | 1 Mol α-C12 | techn. dest. | 8,23 | Triethylendiamin (Diazabicyclooctan) | 5 | Essigsäure | 3,4 | 3,4 | 0,72 | 84,6 | 0,05 | 524 | 4,0 | 11,4 |
| 3 | 1 Mol α-C12 | techn. dest. | 8,23 | Dimethyldodecylamin | 5 | Essigsäure | 1,78 | 2,91 | 0,34 | 94,6 | 0,034 | 527 | 6,0 | 1,7 |
| 4 | 1 Mol α-C12 | techn. dest. | 8,23 | Trioctylamin | 5 | Essigsäure | 1,06 | 81,5 | 8,17 | 13,7 | 0,32 | 419 | 0,2 | 1,0 |
| 5 | 1 Mol α-C12 | techn. dest. | 8,23 | Methylamin, 40%ige Lösg. | 1,63 | Essigsäure | 1,60 | 67,4 | 7,7 | 23,4 | 0,20 | 503 | 5,1 | 1,1 |
| 6 | 1 Mol α-C12 | techn. dest. | 8,23 | Dimethylamin, 40%ige Lösg. | 2,42 | Essigsäure | 1,60 | 5,0 | 0,87 | 89,5 | 0,058 | 534 | 5,7 | 1,9 |
| 7 | 1 Mol α-C12 | techn. dest. | 8,23 | Trimethylamin, 45%ige Lösg. | 2,76 | Essigsäure | 1,60 | 3,4 | 0,67 | 91,5 | 0,085 | 541 | 5,9 | 0,9 |
| 8 | 1 Mol α-C12 | techn. dest. | 8,23 | Dimethyl-(2-hydroxy-dodecyl)-amin | 5,0 | Essigsäure | 1,60 | 3,5 | 0,40 | 92,1 | 0,04 | 539 | 6,2 | 1,2 |
| 9 | 3 Mol α-C8 | dest. | 12,20 | Dimethyl-(2-hydroxy-dodecyl)-Amin | 15,0 | Essigsäure | 4,8 | 1,8 | 6,52 | 81,1 | 0,014 | 730 | 10,5 | 4,4 |
| 10 | 2 Mol α-C14 | techn. dest. | 7,10 | Dimethyl-(2-hydroxy-dodecyl)-Amin | 10 | Essigsäure | 3,2 | 5,2 | 2,0 | 91,3 | 0,0 | 479 | 0,0 | 8,3 |
| 11 | 2 Mol α-C16 | techn. undest. | 6,30 | Dimethyl-(2-hydroxy-dodecyl)-Amin | 10 | Essigsäure | 3,2 | 7,3 | 1,14 | 90,5 | 0,08 | 412 | 0,0 | 7,9 |
| 12 | 2 Mol α-C18 | techn. undest. | 5,49 | Dimethyl-(2-hydroxy-dodecyl)-Amin | 10 | Essigsäure | 3,2 | 12,2 | 1,17 | 87,0 | 0,10 | 369 | 0,0 | 9,5 |

Fortsetzung

| Beispiel | Epoxid Art | Reinh. | %EpO | Katalysator Aminkomponente Art | Menge g | Säurekomponente Art | Menge g | Endprodukte Vorlauf % | %EpO | Hauptlauf % | %EpO | OHZ | Nachl. % | Rückst. % |
|---|---|---|---|---|---|---|---|---|---|---|---|---|---|---|
| 13 | 1 Mol α-C12 | techn. dest. | 8,23 | Trimethylamin, 45%ige Lösg. | 2,76 | Adipinsäure | 1,94 | 64,5 | 8,03 | 18,6 | 0,48 | 528 | 7,31 | 6,32 |
| 14 | 1 Mol α-C12 | techn. dest. | 8,23 | Trimethylamin, 45%ige Lösg. | 2,76 | Laurinsäure | 5,33 | 5,73 | 2,95 | 82,2 | 0,16 | 540 | 9,6 | 4,5 |
| 15 | 1 Mol α-C12 | techn. dest. | 8,23 | Trimethylamin, 45%ige Lösg. | 2,76 | Stearinsäure | 7,56 | 5,04 | 2,83 | 86,4 | 0,13 | 528 | 7,07 | 8,55 |
| 16 | 1 Mol α-C12 | techn. dest. | 8,23 | Trimethylamin, 45%ige Lösg. | 2,76 | techn. Ölsäure | 7,40 | 4,84 | 2,90 | 83,5 | 0,19 | 529 | 11,4 | 5,9 |
| 17 | 1 Mol α-C12 | techn. dest. | 8,23 | Trimethylamin, 45%ige Lösg. | 2,76 | techn. Erucasäure | 9,03 | 5,1 | 2,95 | 83,4 | 0,006 | 534 | 8,75 | 9,0 |
| 18 | 1 Mol α-C12 | techn. dest. | 8,23 | Trimethylamin, 45%ige Lösg. | 2,76 | 85%ige $H_3PO_4$ | 1,3 | 47,0 | 7,5 | 20,2 | 0,32 | 530 | 3,6 | 20,6 |
| 19 | 1 Mol α-C12 | techn. dest. | 8,23 | Dimethyl-2-hydroxy-octylamin | 3,60 | Caprylsäure | 3,80 | 37,9 | 7,5 | 46,3 | 0,06 | 527 | 15,4 | 2,8 |
| 20 | 1 Mol α-C12 | techn. dest. | 8,23 | Dimethyl-2-hydroxy-dodecylamin | 5,12 | Laurinsäure | 5,33 | 34,5 | 7,54 | 49,4 | 0,18 | 532 | 15,3 | 4,1 |
| 21 | 1 Mol α-C12 | techn. dest. | 8,23 | Dimethyl-2-hydroxy-C16/18-alkylamin | 6,73 | Stearinsäure | 7,56 | 45,9 | 7,68 | 36,8 | 0,38 | 520 | 11,8 | 11,0 |
| 22 | 1 Mol α-C12 | techn. dest. | 8,23 | Dimethyl-2-hydroxy-C16/18-alkylamin | 6,73 | Erucasäure | 9,03 | 43,5 | 7,72 | 42,1 | 0,35 | 512 | 7,4 | 11,9 |
| 23 | 1 Mol α-C12 | techn. dest. | 8,23 | Dimethyl-2-hydroxy-dodecylamin | 5,12 | Erucasäure | 9,03 | 34,0 | 7,51 | 48,7 | 0,80 | 534 | 10,3 | 12,1 |
| 24 | 1 Mol α-C12 | techn. dest. | 8,23 | Dimethyl-2-hydroxy-dodecylamin | 5,12 | Stearinsäure | 7,65 | 32,9 | 7,57 | 48,5 | 0,10 | 534 | 18,2 | 5,1 |

Fortsetzung

| Beispiel | Epoxid Art | Reinh. | %EpO | Katalysator Aminkomponente Art | Menge g | Säurekomponente Art | Menge g | Endprodukte Vorlauf % | Vorlauf %EpO | Hauptlauf % | Hauptlauf %EpO | OHZ | Nachl. % | Rückst. % |
|---|---|---|---|---|---|---|---|---|---|---|---|---|---|---|
| 25 | 1 Mol $\alpha$-C12 | techn. dest. | 8,23 | Dimethyldodecylamin | 5,0 | Stearinsäure | 7,56 | 24,7 | 6,66 | 61,7 | 0,05 | 532 | 11,0 | 8,3 |
| 26 | 1 Mol $\alpha$-C12 | techn. dest. | 8,23 | Dimethyl-2-hydroxydodecylamin | 5,12 | Azelainsäure | 5,10 | 42,8 | 8,10 | 42,3 | 0,19 | 532 | 5,6 | 12,0 |
| 27 | 1 Mol $\alpha$-C12 | techn. dest. | 8,23 | Dimethyl-2-hydroxydodecylamin | 5,12 | Sebacinsäure | 5,38 | 39,6 | 7,70 | 45,2 | 0,13 | 538 | 6,9 | 11,8 |
| 28 | 1 Mol $\alpha$-C12 | techn. dest. | 8,23 | Methyl-trioctylammoniumchlorid | 5,0 | Essigsäure | 0,94 | 8,0 | 3,62 | 84,5 | 0,17 | 527 | 10,8 | 1,2 |
| 29 | 1 Mol $\alpha$-C12 | techn. dest. | 8,23 | Dimethyl-bis-(2-hydroxydodecyl)-ammoniumacetat | 5,0 | Essigsäure | 0,17 | 4,6 | 1,76 | 88,8 | 0,06 | 541 | 9,5 | 1,1 |
| 30 | 1 Mol $\alpha$-C12 | techn. dest. | 8,23 | Dimethyl-distearylammoniumchlorid 75%ig | 5,0 | – | 0,0 | 26,7 | 6,73 | 60,0 | 0,22 | 542 | 12,8 | 2,5 |

Beispiel 31

1 Mol Hexenoxid-1 (EpO 15,4%, daraus berechnetes Molgewicht 103,9) wurde mit 1,6 g Essigsäure, 2,42 g 40%iger wäßriger Dimethylaminlösung und 50 g Wasser in einen Rührautoklaven gegeben und 4 Stunden auf 120° C gehalten, wobei sich ein Druck von 7,8 bar einstellte. Die destillative Aufarbeitung ergab 2,6% Vorlauf, 87,4% Hexandiol-1,2 und 1,2% der Theorie. Rückstand. Die Prozentzahlen sind auf das Molgewicht von Hexandiol-1,2 (118,2) bezogen.

Beispiel 32

1 Mol Cetyl-glycidylether (EpO 4,38%, daraus berechnetes Molgewicht 365,3) wurden mit 50 g Wasser, 2,42 g 40%iger wäßriger Dimethylaminlösung und 1,6 g Essigsäure in einen Rührautoklaven gegeben und 6 Stunden auf 135° C erhitzt. Das Endprodukt wurde im Vakuum getrocknet. Die Bestimmung der Hydroxylzahl ergab 284. Die Ausbeute beträgt wenigstens 80%.

Beispiel 33

1/2 Mol Bisphenol-A-diglycidylether (EpO 8,61%, daraus berechnetes Molgewicht 371,8) wurden mit 50 g Wasser, 2,42 g 40%iger Dimethylaminlösung in Wasser und 1,6 g Essigsäure im Rührautoklaven 6 Stunden auf 135° C erhitzt. Das im Vakuum getrocknete Endprodukt besaß die Kennzahlen OHZ 495, EpO 0,14%; Ausbeute wenigstens 80%.

Beispiel 34

1 Mol Styroloxid (EpO 12,53%, daraus berechnetes Molgewicht 127,6) wurde mit 50 g Wasser, 2,42 g 40%iger Dimethylaminlösung in Wasser und 1,6 g Essigsäure im Rührautoklaven 6 Stunden auf 135° C gehalten. Durch Destillation erhielt man 110,9 g (80,2% der Theorie) an Phenylglykol (OHZ 773; EpO 0,0%).

Beispiel 35

Man kocht 1 Mol Dodecenoxid mit 50 g Wasser, 4,84 g 40%iger wäßriger Dimethylaminlösung und 10,7 g Laurinsäure 8 Stunden am Rückfluß, zieht das Wasser im Wasserstrahlvakuum ab und destilliert im Ölpumpenvakuum. Man erhält 177,2 g Dodecandiol, Kp 114—165° C bei 0,1 mbar; OHZ 506 (ber. 555), AZ 8,74; SZ 0,27 (Ausbeute 87,6% der Theorie).

Beispiel 36

1 Mol Hexenoxid wird mit 50 g Wasser, 2,42 g 40%iger wäßriger Dimethylaminlösung und 1,6 g Essigsäure in einen Autoklaven gegeben und 4 Stunden auf 120° C erhitzt. Nach Abdestillieren des Wassers im Wasserstrahlvakuum wird auch das Endprodukt im Wasserstrahlvakuum bei 12 Torr destilliert. Man erhält 103 g (87% der Theorie) Hexandiol, $Kp_{12}$ 114—145° C; OHZ 901 (ber. 949).

Beispiel 37

1 Mol Dodecenoxid, 50 g Wasser, 2,42 g 40%ige wäßrige Dimethylaminlösung, 4,1 g Zitronensäure werden 4 Stunden unter autogenem Druck auf 160° C gehalten. Man erhält 183,1 g (90,5% der Theorie) an Diol.

Wird anstelle der Zitronensäure Benzoesäure eingesetzt, so erhält man nach 4 Stunden bei 120° C 68,6% der Theorie an gewünschtem Diol.

Die Verwendung von p-Aminobenzoesäure führt im gleichen Versuch bei 4 Stunden und 120° C zur Diolausbeute von 46,2% der Theorie. Bei Verschärfung der Reaktionsbedingungen auf 6 Stunden bei 160° C entsteht das Diol mit einer Ausbeute von 81% der Theorie.

**Patentansprüche**

1. Verfahren zur Herstellung von 1,2-Diolen mit mindestens 4 Kohlenstoffatomen durch Spaltung der entsprechenden 1,2-Epoxidverbindungen mit Wasser in Gegenwart von Katalysatoren bei erhöhten

Temperaturen, dadurch gekennzeichnet, daß man als Katalysatoren Salze von primären, sekundären und/oder tertiären Aminen mit organischen und/oder anorganischen Säuren und/oder quartäre Ammoniumsalze organischer und/oder anorganischer Säuren einsetzt.

2. Verfahren nach Anspruch 1, dadurch gekennzeichnet, daß man mit Aminsalzen von ein- oder mehrbasischen Carbonsäuren als Katalysator arbeitet.

3. Verfahren nach den Ansprüchen 1 und 2, dadurch gekennzeichnet, daß man Katalysatoren einsetzt, die die Säurekomponenten in stöchiometischem Überschuß, vorzugsweise in leichtem Überschuß, über die zur Salzbildung benötigte Menge enthalten.

4. Verfahren nach Anspruch 3, dadurch gekennzeichnet, daß man Katalysatoren einsetzt, die auf eine basische Gruppe 1—2 salzbildende saure Gruppen enthalten, wobei vorzugsweise das stöchiometrische Verhältnis von Base zu Säure 1 : 1,1 bis 1,5 beträgt.

5. Verfahren nach Ansprüchen 1—4, dadurch gekennzeichnet, daß man mit Aminen als Katalysatorkomponente arbeitet, deren Kohlenstoffzahl auf die Kohlenstoffzahl der eingesetzten Epoxidverbindungen wie folgt abgestimmt worden ist:

a) bei einem primären Amin:
Die Summe der Kohlenstoffatome des primären Amins plus die dreifache Summe der Kohlenstoffatome des Epoxids beträgt 8 bis 40, vorzugsweise 12 bis 30;

b) bei einem sekundären Amin:
Die Summe der Kohlenstoffatome des sekundären Amins plus die doppelte Summe der Kohlenstoffatome des Epoxids beträgt 8 bis 40, vorzugsweise 12 bis 30;

c) bei einem tertiären Amin:
Die Summe der Kohlenstoffatome des tertiären Amins plus die Summe der Kohlenstoffatome des Epoxids beträgt 8 bis 40, vorzugsweise 12 bis 30.

6. Verfahren nach Ansprüchen 1—5, dadurch gekennzeichnet, daß man mit — gewünschtenfalls substituierten — aliphatischen, araliphatischen und gegebenenfalls aromatischen Aminen und dabei bevorzugt mit sekundären Aminen als Aminkomponente arbeitet.

7. Verfahren nach Ansprüchen 1—6, dadurch gekennzeichnet, daß man mit Dialkylaminen mit bis zu 8 Kohlenstoffatomen, vorzugsweise mit bis zu 4 Kohlenstoffatomen als Aminkomponente arbeitet und insbesondere Dimethylaminsalze einsetzt.

8. Verfahren nach Ansprüchen 1—7, dadurch gekennzeichnet, daß man zur Salzbildung im Katalysator Mono- und/oder Polycarbonsäuren mit bis zu 26 Kohlenstoffatomen, vorzugsweise Mono- und/oder Dicarbonsäuren mit 2 bis 18 Kohlenstoffatomen einsetzt.

9. Verfahren nach Ansprüchen 1—8, dadurch gekennzeichnet, daß man das Aminsalz in Mengen von 0,5 bis 10 Molprozent, vorzugsweise 1 bis 6 Molprozent, bezogen auf eingesetztes Epoxid, verwendet.

10. Verfahren nach Ansprüchen 1—9, dadurch gekennzeichnet, daß man Epoxidverbindungen mit 4—30 Kohlenstoffatomen, vorzugsweise 6—20 Kohlenstoffatomen, einsetzt, wobei ein die 1,2-Epoxygruppe enthaltender Molekülbestandteil auch über eine Ethergruppierung — vorzugsweise als Glycidylether — an den Kohlenwasserstoffrest gebunden sein kann.

11. Verfahren nach Ansprüchen 1—10, dadurch gekennzeichnet, daß bei Temperaturen bis 180° C, vorzugsweise bei Temperaturen von 100—160° C, gearbeitet wird.

12. Verfahren nach Ansprüchen 1—11, dadurch gekennzeichnet, daß bei Drucken von 1—10 bar, vorzugsweise bei Drucken von 1—6 bar, gearbeitet wird.

13. Verfahren nach Ansprüchen 1—12, dadurch gekennzeichnet, daß bis zu 10 Mol Wasser, vorzugsweise bis zu 5 Mol Wasser, je Mol Epoxidverbindung eingesetzt werden.

14 Verfahren nach Ansprüchen 1—13, dadurch gekennzeichnet, daß die thermisch-hydrolytische Behandlung der Epoxidverbindungen für die Dauer von 2 bis 10 Stunden, vorzugsweise unter Bewegen des Reaktionsgemisches zum Beispiel durch Rühren vorgenommen wird.

## Claims

1. A process for the production of 1,2-diols containing at least 4 carbon atoms by splitting the corresponding 1,2-epoxide compounds with water in the presence of catalysts at elevated temperatures, characterised in that the catalysts used are salts of primary, secondary and/or tertiary amines with organic and/or inorganic acids and/or quaternary ammonium salts of organic and/or inorganic acids.

2. A process as claimed in Claim 1, characterised in that amine salts of mono- or polybasic carboxylic acids are used as the catalyst.

3. A process as claimed in Claims 1 and 2, characterised in that the catalysts used contain the acid components in a stoichiometric excess and preferably in a slight excess over and above the quantity required for salt formation.

4. A process as claimed in Claim 3, characterised in that the catalysts used contain 1 to 2 salt-forming acid groups to one basic group, the stoichiometric ratio of base to acid preferably amounting to

beween 1 : 1.1 and 1 : 1.5.

5. A process as claimed in Claims 1 to 4, characterised in that the catalysts used are amines of which the number of C-atoms has been adapted as follows to the number of C-atoms in the epoxide compounds used:

a) in the case of a primary amine:
the sum of the carbon atoms in the primary amine plus three times the sum of the carbon atoms in the epoxide amounts to between 8 and 40 and preferably to between 12 and 30;
b) in the case of a secondary amine:
the sum of the carbon atoms in the secondary amine plus twice the sum of the carbon atoms in the epoxide amounts to between 8 and 40 and preferably to between 12 and 30;
c) in the case of a tertiary amine:
the sum of the carbon atoms in the tertiary amine plus the sum of the carbon atoms in the epoxide amounts to between 8 and 40 and preferably to between 12 and 30.

6. A process as claimed in Claims 1 to 5, characterised in that aliphatic, araliphatic and optionally aromatic — if desired substituted — amines, preferably secondary amines, are used as the amine component.

7. A process as claimed in Claims 1 to 6, characterised in that dialkylamines containing up to 8 C-atoms and preferably up to 4 C-atoms, more particularly dimethyl; amine salts, are used as the amine component.

8. A process as claimed in Claims 1 to 7, characterised in that mono- and/or polycarboxylic acids containing up to 26 C-atoms, preferably mono- and/or dicarboxylic acids containing from 2 to 18 C-atoms, are used for salt formation in the catalyst.

9. A process as claimed in Claims 1 to 8, characterised in that the amine salt is used in quantities of from 0.5 to 10 mole percent and preferably in quantities of from 1 to 6 mole percent, based on the epoxide used.

10. A process as claimed in Claims 1 to 9, characterised in that epoxide compounds containing from 4 to 30 C-atoms and preferably from 6 to 20 C-atoms are used, in which case a constituent of the molecule containing the 1,2-epoxy group may be attached to the hydrocarbon radical through an ether group, preferably as glycidyl ether.

11. A process as claimed in Claims 1 to 10, characterised in that it is carried out at temperatures of up to 180° C and preferably at temperatures in the range from 100 to 160° C.

12. A process as claimed in Claims 1 to 11, characterised in that it is carried out under pressures of from 1 to 10 bar and preferably under pressures of from 1 to 6 bar.

13. A process as claimed in Claims 1 to 12, characterised in that up to 10 moles of water and preferably up to 5 moles of water are used per mole of epoxide compound.

14. A process as claimed in Claims 1 to 13, characterised in that the thermal-hydrolytic treatment of the epoxide compound is carried out for 2 to 10 hours during which the reaction mixture is preferably kept in motion, for example by stirring.

**Revendications**

1. Procédé de fabrication de diols-1,2 ayant au moins 4 atomes de carbone par clivage des composés époxy-1,2 correspondants avec de l'eau en présence de catalyseurs à des températures élevées, caractérisé en ce qu'on utilise comme catalyseurs des sels d'amines primaires, secondaires et/ou tertiaires avec des acides organiques et/ou minéraux, et/ou des sels d'ammonium quaternaires d'acides organiques et/ou minéraux.

2. Procédé selon la revendication 1, caractérisé en ce qu'on opère avec des sels d'amines d'acides carboxyliques mono- ou polybasiques comme catalyseur.

3. Procédé selon les revendications 1 et 2, caractérisé en ce qu'on utilise des catalyseurs qui contiennent les composants acides en excès stoechiométrique, de préférence en léger excès par rapport à la quantité nécessaire pour la formation du sel.

4. Procédé selon la revendication 3, caractérisé en ce qu'on utilise des catalyseurs qui contiennent pour un groupe basique 1—2 groupes acides formateurs de sels, le rapport stoechiométrique base/acide s'élèvant de préférence à 1 : 1,1 à 1,5.

5. Procédé selon les revendications 1 à 4, caractérisé en ce qu'on opère avec des amines comme composant catalyseur dont le nombre d'atomes de carbone a été formulé de la manière suivante par rapport au nombre d'atomes de carbone des composés époxy mis en jeu:

a) pour une amine primaire:
la somme des atomes de carbone de l'amine primaire plus la somme triple des atomes de carbone de l'époxyde s'élève à 8—40, de préférence à 12—30;
b) pour une amine secondaire:

la somme des atomes de carbone de l'amine secondaire plus la somme double des atomes de carbone de l'époxyde s'élève à 8—40, de préférence à 12—30;

c) pour une amine tertiaire:
la somme des atomes de carbone de l'amine tertiaire plus la somme des atomes de carbone de l'époxyde s'élève à 8—40, de préférence à 12—30.

6. Procédé selon les revendications 1 à 5, caractérisé en ce qu'on opère avec des amines — éventuellement substituées — aliphatiques, araliphatiques et éventuellement aromatiques, de préférence ici avec des amines secondaires comme composant aminé.

7. Procédé selon les revendications 1 à 6, caractérisé en ce qu'on opère avec des dialcoylamines ayant jusqu'à 8 atomes de carbone, de préférence jusqu'à 4 atomes de carbone comme composant aminé et en ce qu'on utilise en particulier des sels de diméthylamine.

8. Procédé selon les revendications 1 à 7, caractérisé en ce qu'on utilise pour la formation du sel dans le catalyseur des acides mono- et/ou polycarboxyliques aynat jusqu'à 26 atomes de carbone, de préférence des acides mono- et/ou dicarboxyliques ayant 2 à 18 atomes de carbone.

9. Procédé selon les revendications 1 à 8, caractérisé en ce qu'on utilise le sel d'amine en des quantités de 0,5 à 10 moles %, de préférence de 1 à 6 moles % par rapport à l'époxyde employé.

10. Procédé selon les revendications 1 à 9, caractérisé en ce qu'on utilise des composés époxy ayant 4—30 atomes de carbone, de préférence 6—20 atomes de carbone, cas où un constituant moléculaire contenant le groupe époxy-1,2 peut aussi être relié au radical hydrocarboné par l'intermédiaire d'un groupe éther — de préférence sous la forme de glycidyl-éther.

11. Procédé selon les revendications 1 à 10, caractérisé en ce qu'on opère à des températures allant jusqu'à 180° C, de préférence à des températures de 100 à 160° C.

12. Procédé selon les revendications 1 à 11, caractérisé en ce qu'on opère sous des pressions de 1 à 10 bars, de préférence sous des pressions de 1—6 bars.

13. Procédé selon les revendications 1 à 12, caractérisé en ce qu'on utilise jusqu'à 10 moles d'eau, de préférence jusqu'à 5 moles d'eau par mole de composé époxy.

14. Procédé selon les revendications 1 à 13, caractérisé en ce que le traitement thermohydrolytique des composés époxy est entrepris pendant une durée de 2 à 10 heures, de préférence en remuant le mélange de réaction par exemple par agitation.